# FASCICULE DE BREVET EUROPEEN

(11) **EP 2 997 996 B1**
(45) Date de publication et mention de la délivrance du brevet: **09.01.2019**
(21) Numéro de dépôt: 15184989.0
(22) Date de dépôt: 14.09.2015
(51) Int. Cl.: A61N 1/05, A61B 5/00, A61B 5/02, A61B 5/021, A61B 5/0295, A61B 5/053, A61N 1/36

(54) **DISPOSITIF IMPLANTABLE COMPRENANT UNE SONDE DE STIMULATION PERMETTANT UNE MESURE DE BIOIMPÉDANCE SUR UN FLUX SANGUIN**
IMPLANTIERBARE VORRICHTUNG, DIE EINE STIMULATIONSSONDE ZUR MESSUNG DER BIOIMPEDANZ IN EINEM BLUTSTROM UMFASST
IMPLANTABLE DEVICE COMPRISING A STIMULATION PROBE ALLOWING A BIOIMPEDANCE MEASUREMENT ON A BLOOD FLOW

(30) Priorité: 19.09.2014 FR 1458857
(43) Date de publication de la demande: 23.03.2016
(73) Titulaire: Sorin CRM SAS, 92140 Clamart Cedex (FR)
(72) Inventeur: BONNET, Jean-Luc, 91300 Massy (FR); LEGAY, Thierry, 91640 Fontenay les Briis (FR); DECOENE, Dominique, 78760 Jouars Pontchartrain (FR); LE GOUSSE, Patrick, 91170 Viry Chatillon (FR); COLLIGNON, Mathieu, 91460 Marcoussis (FR)
(74) Mandataire: Grünecker Patent- und Rechtsanwälte PartG mbB

(56) Documents cités:
- EP-A2- 1 363 697
- WO-A1-2013/022886
- US-A1- 2011 009 754
- US-A1- 2014 121 556

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus précisément la mesure des paramètres hémodynamiques.

La mesure de ces paramètres est un élément clé de la stimulation cardiaque. Les paramètres hémodynamiques peuvent inclure la pression artérielle, mesurée par un capteur de pression (par exemple à jauge de contrainte), la contractilité cardiaque (mesurée par exemple par un accéléromètre) et les volumes cardiaques (mesurés par exemple par un capteur de conductance dans le ventricule gauche).

Ces mesures permettent d'informer le dispositif de stimulation sur l'effet d'une thérapie telle qu'une stimulation cardiaque ou une neurostimulation au niveau du nerf vague.

Par ailleurs, dans le cadre de telles stimulations, il peut être utile de mesurer et d'enregistrer des informations sur l'état physiologique du patient, telles que l'activité cardiaque et cardiovasculaire, l'activité respiratoire ou encore l'activité gastrique. Ceci permet d'une part d'appliquer la stimulation en synchronisme avec un ou plusieurs paramètres, et d'autre part de surveiller l'efficacité de la thérapie par stimulation.

Par exemple, pour des patients souffrant d'insuffisance cardiaque (typiquement une fraction d'éjection du ventricule gauche LVEF trop faible dans le cas d'un coeur dilaté, ou une réduction des volumes cardiaques à LVEF constante dans le cas d'un coeur hypertrophié), la fonction cardiaque peut être rendue plus efficace par une stimulation du nerf vague. En outre, il est connu que l'insuffisance cardiaque est souvent associée à l'hypertension artérielle.

On a déjà proposé différentes solutions pour mesurer les paramètres hémodynamiques liés à l'activité cardiaque, notamment afin d'ajuster les paramètres d'une stimulation cardiaque.

L'utilisation connue d'un capteur de pression ou d'un accéléromètre dans un dispositif implanté à long terme est susceptible de poser de nombreux problèmes : durabilité, nécessité d'une sonde dédiée, consommation électrique.

Quant aux systèmes basés sur la mesure de la bioimpédance, ils nécessitent une sonde multipolaire spécifique et doivent être placés dans le coeur ou à proximité de celui-ci.

Une autre technique est décrite par le WO 2013/022886 A1, consistant à mesurer de paramètres hémodynamiques par injection d'un courant dans un organe d'une région du corps au niveau cervical et recueil des variations de tensions induites à proximité, par exemple pour évaluer les variations instantanées du volume d'une artère par une mesure d'impédance (pléthysmographie par impédance). Dans une configuration monopolaire, l'impédance est évaluée entre le boitier du générateur implanté et une électrode placée sur le nerf vague, mais cette configuration ne permet pas d'obtenir une mesure de l'impédance locale représentative du débit sanguin dans l'artère carotide. Dans une autre configuration, bipolaire, ces variations locales peuvent être mesurées, mais toute variation de l'impédance de contact de l'électrode au point d'injection du courant est susceptible d'induire une variation de tension parasite, non représentative du débit sanguin ; cette variation est en outre susceptible de créer dans le signal des sauts de tension pouvant saturer les étages amplificateurs du circuit de détection du dispositif.

La présente invention vise à proposer un nouveau capteur de bioimpédance capable d'effectuer différents types de mesures d'impédance avec une sonde unique, pouvant être aisément intégré à une sonde dédiée à une autre fonction, telle qu'une fonction de stimulation.

L'invention vise spécifiquement le problème consistant à fournir des signaux non affectés par d'éventuelles variations locales de l'impédance de contact, donc des signaux reflétant précisément les variations du paramètre hémodynamique mesuré, au surplus sans risque de saturation pour les circuits de détection du dispositif.

Cette fonction de mesure d'une bioimpédance par injection de courant, avec les problèmes qui lui sont propres, doit être distinguée de celle consistant à délivrer des impulsions de stimulation (ou recueillir des signaux produits par un organe), qui fait appel à des électrodes distinctes et dont la configuration est spécifique à la fonction de stimulation/détection. Le EP 1 363 697 A2 (publié sous la référence WO 02/18006 A2) décrit ainsi une sonde de détection/stimulation des cavités cardiaques, implantable dans un vaisseau coronaire. Mais s'agissant d'appliquer des impulsions de tension, ou de détecter des potentiels de dépolarisation (recueil d'un électrogramme), les variations éventuelles d'une l'impédance de contact au point d'injection d'un courant ne se pose pas.

Plus précisément, l'invention propose un dispositif implantable comprenant, de manière en elle-même connue notamment d'après le WO 2013/022886 A1 précité, une sonde de stimulation destinée à être placée dans une région corporelle à stimuler électriquement, cette sonde comprenant une série d'électrodes de stimulation reliées à un dispositif de commande, et une série d'électrodes aptes à effectuer des mesures de bioimpédance sur un flux sanguin situé dans ladite région.

La série d'électrodes comprend une première paire d'électrodes de mesure de bioimpédance reliées à un générateur de courant pour faire circuler un courant de façon contrôlée dans le vaisseau sanguin, et une deuxième paire d'électrodes de mesure de bioimpédance reliées à l'entrée d'un circuit d'amplification et de traitement, et configurées de manière à capter un signal à partir duquel peuvent être extraites lesdites mesures de bioimpédance, ce signal étant engendré en réponse audit courant.

De façon caractéristique de l'invention, la première et la deuxième paire d'électrodes de mesure de bioimpédance et la deuxième paire d'électrodes de mesure de bioimpédance partagent une électrode commune (E1) formant électrode de référence.

Selon diverses caractéristiques subsidiaires avantageuses :
- les électrodes de mesure de bioimpédance sont agencées de façon affleurante à la surface d'un corps de la sonde ;
- les électrodes de mesure de bioimpédance sont alignées parallèlement à une direction axiale de la sonde ;
- le circuit d'amplification et de traitement comprend des moyens de séparation en bandes de fréquences, comprenant notamment des moyens aptes à déterminer des variations d'amplitude du signal dans les différentes bandes de fréquences ;
- ces bandes de fréquences sont choisies pour refléter au moins deux activités parmi une activité vasomotrice, une activité respiratoire, une activité hémodynamique et une activité du rythme cardiaque, une bande de fréquences pouvant être choisie pour déterminer des variations de la pression sanguine sur un cycle cardiaque ;
- la sonde est configurée pour appliquer une neurostimulation au nerf vague, et les électrodes de mesure de bioimpédance sont configurées pour longer un vaisseau s'étendant généralement dans la même direction que le nerf vague, notamment pour longer l'artère carotide, les paires d'électrodes de mesure de bioimpédance étant alors séparées d'une distance comprise entre 10 et 25 mm environ au sein d'une paire, avantageusement avec une superficie d'électrodes comprise entre environ 0,5 et 2 mm²;
- le dispositif comprend en outre un circuit de commande apte à contrôler la neurostimulation en fonction des mesures de bioimpédance effectuées, et comprend alors avantageusement un boitier implantable relié à la sonde par une liaison comprenant des conducteurs pour la neurostimulation et des conducteurs pour les signaux de bioimpédance de la sonde vers le boitier ;
- les électrodes de la deuxième paire d'électrodes de mesure de bioimpédance sont configurées de manière à capter ledit signal à partir duquel peuvent être extraites lesdites mesures de bioimpédance de façon indépendante d'une impédance de contact au point d'injection dudit courant.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 illustre schématiquement la configuration des nerfs vagues, des artères carotides et des veines jugulaires dans la région du cou d'un être humain.
La Figure 2 illustre schématiquement un positionnement possible d'un dispositif implanté comportant une sonde capable de participer à une mesure impédancemétrique.
La Figure 3 illustre schématiquement une sonde de neurostimulation placée autour du nerf vague.
La Figure 4 illustre schématiquement la partie de détection impédancemétrique d'une telle sonde.
La Figure 5 illustre schématiquement un circuit d'injection de courant et un circuit de lecture de courant pour la mesure impédancemétrique.
La Figure 6 représente schématiquement une configuration d'électrodes de la sonde des Figures 3 et 4.
La Figure 7 représente plus en détail un circuit d'injection de courant et de traitement associé aux électrodes.
La Figure 8 représente une version améliorée du circuit de traitement de la Figure 7.
La Figure 9 représente des échantillons d'un signal d'impédancemétrie recueilli.
La Figure 10 est une représentation analogique d'un signal recueilli sur une période sensiblement plus longue.
La Figure 11 représente différents pics de signaux obtenus après traitement du signal.
La Figure 12 illustre certains paramètres d'un signal de pression sanguine dérivé du signal recueilli.
La Figure 13 est un schéma par blocs illustrant les différentes bandes de fréquences et les fonctions vitales associées.
La Figure 14 est un schéma par blocs illustrant l'intégration du dispositif de mesure et de traitement impédancemétrique à une sonde de neurostimulation.

On va maintenant décrire un exemple de réalisation du dispositif de l'invention.

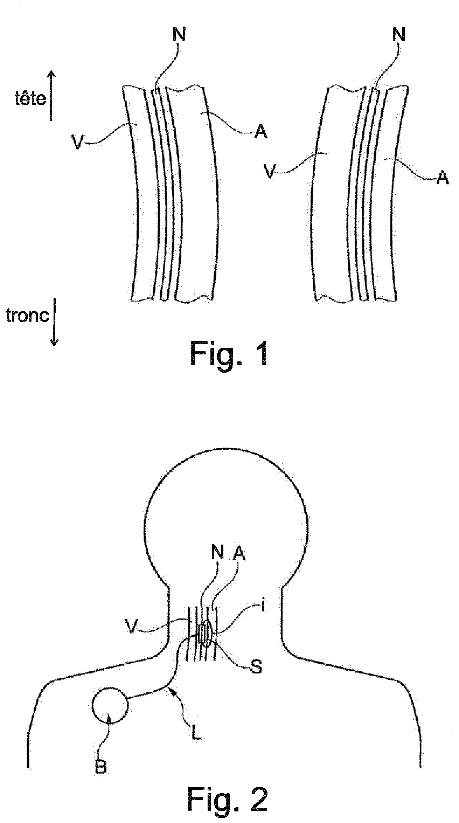

En référence tout d'abord à la Figure 1, on a représenté dans la région cervicale du corps humain les deux ensembles (veine jugulaire V, nerf vague N, artère carotide A), respectivement gauche et droit.

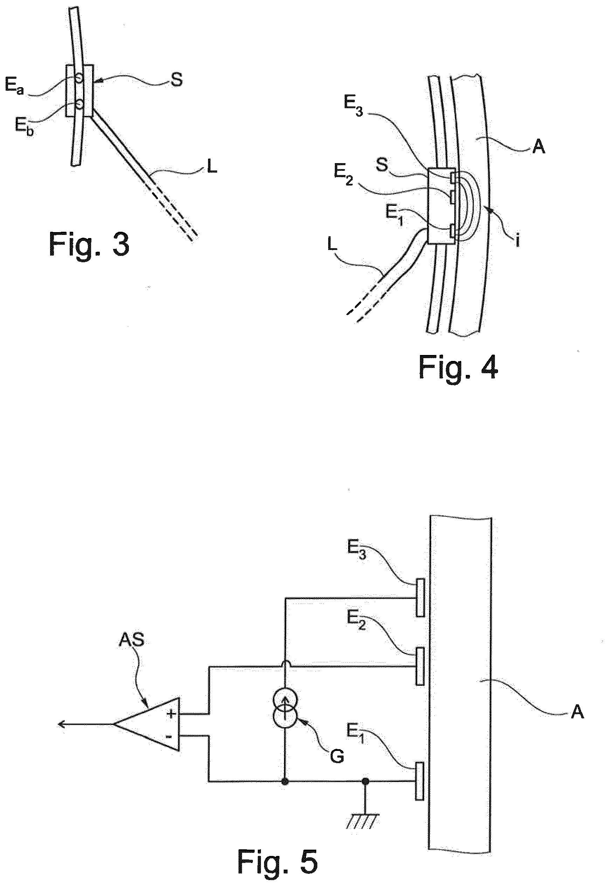

En référence à la Figure 2, une sonde S de stimulation du nerf vague N est implantée et positionnée autour du nerf vague, de préférence au niveau cervical. Elle est reliée à un boitier implanté B par une liaison L. La Figure 3 illustre schématiquement le positionnement de la sonde S autour du nerf vague. Elle est équipée d'un ensemble d'électrodes de stimulation, typiquement un système bipolaire comprenant les électrodes Ea, Eb. Dans d'autres applications, le système peut être quasi-tripolaire, tripolaire ou multipolaire, cela ne changeant pas le principe même de la présente invention.

La sonde S de stimulation du nerf vague N est également utilisée comme capteur d'impédance au niveau de l'artère carotide A, de manière à déterminer des variations d'impédance, dans différentes bandes de fréquences, reflétant elles-mêmes les variations du flux sanguin artériel.

La Figure 4 illustre en détail le positionnement sur le nerf vague de la sonde S, qui est, selon l'invention, une sonde tripolaire pourvue de trois électrodes E1, E2, E3, de préférence différentes de Ea et Eb, pour l'acquisition impédancemétrique, et différents trajets de courant i dans l'artère A entre deux électrodes E1, E3, grâce auquel une impédance dynamique dans l'artère va pouvoir être mesurée.

En référence à la Figure 5, on a représenté les trois électrodes E1, E2 et E3 de mesure d'impédance faisant partie de la sonde de stimulation S et situées au voisinage de l'écoulement sanguin dans l'artère carotide A, dans un alignement généralement parallèle à la direction de cet écoulement. Cet ensemble d'électrodes comprend une électrode de référence E1, une électrode d'injection de courant E3 et une électrode de mesure E2 située entre les deux électrodes E1 et E3. Les électrodes E1 et E3 sont alimentées par un générateur de courant G, tandis que l'électrode intermédiaire de mesure E2, située plus près de l'électrode E3 que de l'électrode E1, est reliée, avec ladite électrode E1, aux entrées d'un amplificateur de sortie AS.

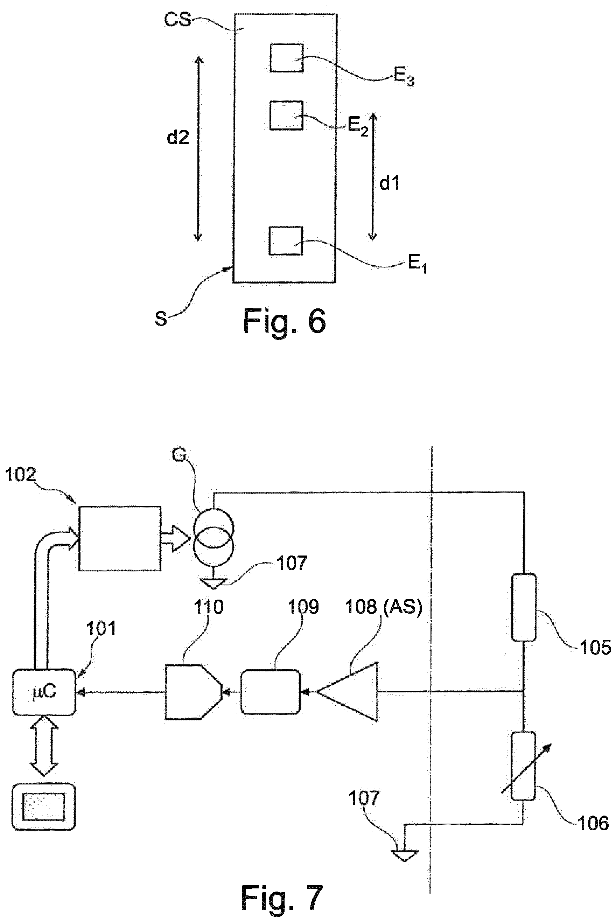

De préférence, et comme le montrent les Figures 4 et 6, les électrodes E1 à E3 sont réalisées en affleurement dans un corps CS de la sonde S, réalisé en matière isolante biocompatible, typiquement un silicone, de manière à faire face à l'artère carotide A.

Ce corps présente typiquement une longueur de l'ordre de 25 mm. Les électrodes E1 et E3 sont par exemple espacées d'une distance de l'ordre de 20 mm, tandis que la distance entre les électrodes E1 et E2 est de l'ordre de 15 mm. Plus généralement, la distance entre les électrodes E2 et E3 correspond avantageusement à une fraction de la distance entre les électrodes E1 et E3 comprise entre 5 et 50 %, de préférence le pourcentage le plus faible possible, afin de maximiser le champ électrique recueilli.

Les électrodes présentent une surface exposée préférentiellement comprise entre 0,5 et 2 mm² environ.

La Figure 7 illustre un circuit de mesure d'impédance utilisé avec la sonde décrite ci-dessus.

Le circuit comprend un microcontrôleur 101 qui pilote un circuit de commande d'injection de courant 102 agissant sur la source de courant G reliée à l'électrode E3 et à l'électrode E1 via la masse 107. Un courant bien défini est ainsi injecté entre les électrodes E1 et E3. Ce courant est préférentiellement impulsionnel à une fréquence entre 8 et 128 Hz, les impulsions ayant une amplitude de 30 µA à 1000 µA pour une largeur de 5 µs à 50 µs.

Les tissus dans lesquels circule ce courant peuvent être assimilés à une bioimpédance statique, désignée par la référence 105, en série avec une bioimpédance variable, désignée par la référence 106.

La mesure est effectuée entre les électrodes E1 et E2, et l'étage de mesure comprend l'amplificateur de tension 108 (l'amplificateur AS de la Figure 5) dont la sortie est reliée à l'entrée d'un convertisseur analogique/numérique 110 via un circuit de filtrage analogique 109. La sortie de ce convertisseur est appliquée sur une entrée du microcontrôleur 101 pour traitement.

On notera que l'on dispose ainsi, de façon caractéristique de l'invention, d'un troisième point de contact (E2) dédié au recueil du signal à amplifier et distinct du point d'injection du courant (E3). Dès lors, cette configuration tripolaire locale présente l'avantage, propre à l'invention, que l'impédance de contact présente au point d'injection du courant est sans effet sur la mesure du signal, et que les variations éventuelles de cette impédance de contact n'affecteront pas le recueil de la tension à mesurer, du fait de l'impédance d'entrée élevée de l'étage amplificateur AS.

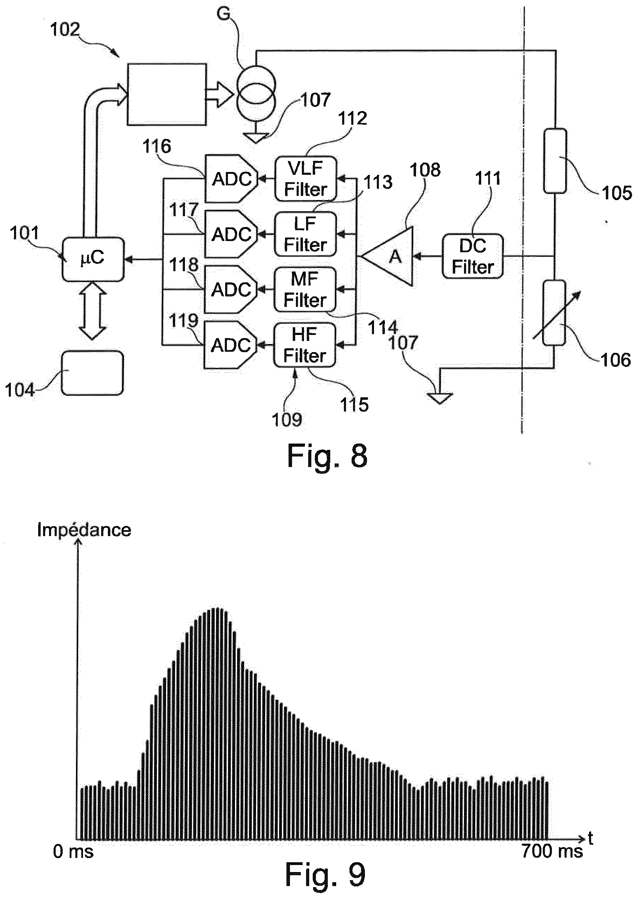

Dans une forme de réalisation préférée, et en référence à la Figure 8, le circuit de filtrage 109 comprend une batterie de filtres passe-bande sur différentes plages de fréquences, ici quatre filtres, permettant de séparer le signal reçu en canaux fréquentiels distincts. Ces filtres sont désignés par les références 112 à 115.

La sortie de chaque filtre est reliée à l'entrée d'un convertisseur analogique/numérique respectif (références 116 à 119), ceux-ci pouvant être réalisés par multiplexage des différents canaux dans un convertisseur unique.

Selon une caractéristique avantageuse, les fréquences d'échantillonnage des différents convertisseurs 116 à 119 sont adaptées aux différentes bandes de fréquences créées par les filtres 112 à 115.

Comme le montre également la Figure 8, un filtre coupe-bas 111 peut être prévu en amont de l'amplificateur 108 pour éliminer la composante continue du signal recueilli au niveau des électrodes E1 et E2.

Selon une autre variante, on peut appliquer la sortie de l'amplificateur 108 directement à l'entrée d'un convertisseur analogique/numérique unique 110, les traitements de filtrage étant mis en oeuvre par filtrage numérique au sein du microcontrôleur 101, ou dans un processeur de signal numérique (DSP) dédié 104 associé au microcontrôleur.

La Figure 9 illustre, sur un cycle cardiaque, l'évolution des échantillons du signal d'impédance brut recueilli, dont on observe qu'il est bien représentatif de la pression sanguine.

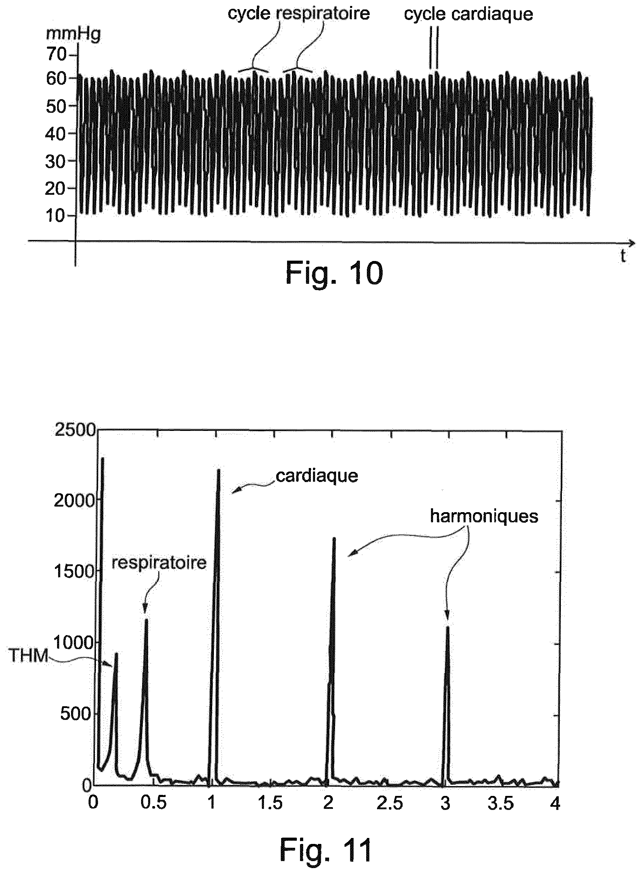

La Figure 10 illustre l'extraction, à partir du signal recueilli, d'un signal de pression ventriculaire sur la fréquence cardiaque. On décèle sur cette figure une modulation de l'amplitude du signal de pression ventriculaire, qui est associée au cycle respiratoire (ici une oscillation sur six cycles cardiaques).

On comprend donc que par une discrimination fréquentielle par filtrages sélectifs comme décrit plus haut, il est possible de déduire du signal, de façon discriminante, des évolutions dans différentes plages de fréquences. Notamment :
- le signal dans de très basses fréquences (typiquement de l'ordre de 0,1 à 0,2 Hz) contient des variations vasomotrices, dénommées également ondes de Traube-Hering-Mayer ou THM ;
- le signal dans de très basses à basses fréquences (typiquement de 0,01 à 0,5 Hz) contient des variations liées à la fonction respiratoire ;
- le signal dans des fréquences moyennes (typiquement de 1 à 3 Hz) contient des variations liées à la fonction cardiaque ;
- enfin, le signal dans des hautes fréquences (typiquement jusqu'à 50 Hz) contient des variations hémodynamiques.

La Figure 11 illustre l'application d'une transformation de Fourier rapide (FFT) sur le signal brut pour déterminer ses différentes composantes fréquentielles. On observe un pic lié aux ondes THM, un pic lié à la fonction respiratoire, un pic lié à la fonction cardiaque ainsi que des harmoniques liées aux variations hémodynamiques, dans l'ordre des fréquences croissantes.

Le système peut effectuer des déductions relatives à l'effet d'une thérapie, ou à un diagnostic, non seulement par la hauteur et la largeur de tels pics, mais également sur la base des formes d'onde associées.

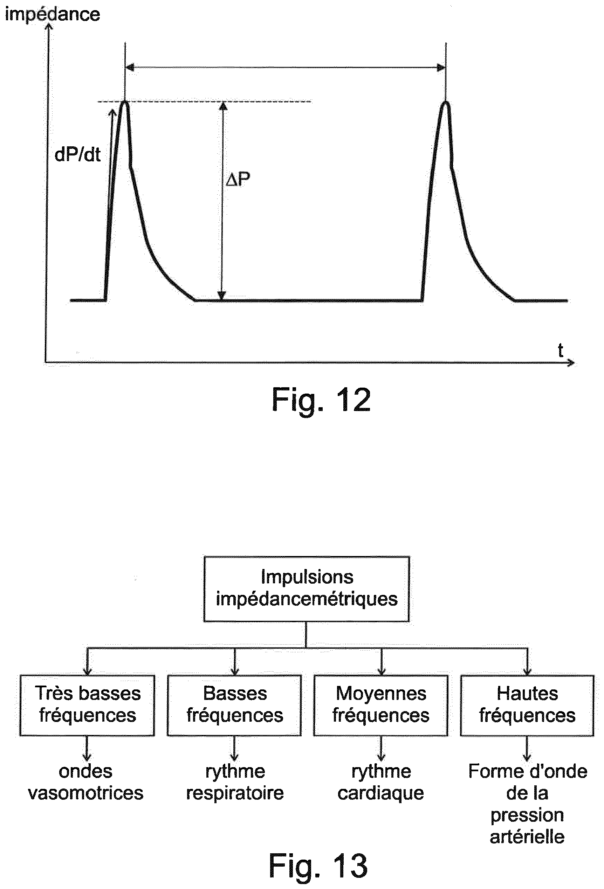

Ainsi, la Figure 12 illustre la possibilité de déterminer entre autres la vitesse de montée en pression lors de la phase systolique du ventricule (dP/dt), et la différence entre les pressions systolique et diastolique (ΔP). La Figure 13 illustre sous forme de schéma par blocs l'analyse multibande décrite dans ce qui précède, avec quelques exemples de paramètres extraits. L'invention permet notamment de mesurer les paramètres suivants, selon les bandes de fréquences :
- rythme cardiaque
- rythme respiratoire,
- dérivée maximale de la pression par rapport au temps lors de la contraction ventriculaire,
- variations des ondes THM sur le long terme, révélatrices de variations de la fonction vasomotrice.

Dans une application de la présente invention, l'activité systolique est extraite du signal d'impédance pour adapter la délivrance de la thérapie de neurostimulation. L'avantage de ce dispositif est d'éviter le recours à une sonde dédiée à la détection de l'activité cardiaque, telle qu'une sonde placée dans le ventricule droit).

Dans certaines formes de mise en oeuvre, il est possible de configurer un dispositif implantable (par exemple une sonde) pour lui permettre d'effectuer des mesures de bioimpédance sur un flux sanguin dans un vaisseau. Dans certains cas, le dispositif implantable peut comprendre un support de stockage informatique (par exemple une mémoire) contenant des instructions qui, lorsqu'elles sont exécutées, font en sorte que le dispositif implantable exécute diverses étapes du procédé.

Dans d'autres cas, le dispositif implantable peut faire passer dans un premier jeu d'électrodes un courant généré par une source de courant. Ce courant peut être généré de façon simplement contrôlée (c'est-à-dire avec une valeur maintenue dans une plage de tolérance donnée). Le dispositif implanté peut alors mesurer un signal en réponse au courant en utilisant un second jeu d'électrodes. Ce signal peut alors être utilisé pour déterminer la bioimpédance mesurée. Le second jeu d'électrodes peut être éventuellement relié à une entrée d'un circuit d'amplification et de traitement. Le premier et le second jeu d'électrodes peuvent partager une référence commune. Dans certains cas, le signal peut être en outre utilisé pour déterminer un ou plusieurs paramètres tels que fréquence cardiaque, fréquence respiratoire, variations de la pression sanguine, variations des ondes THM, etc.

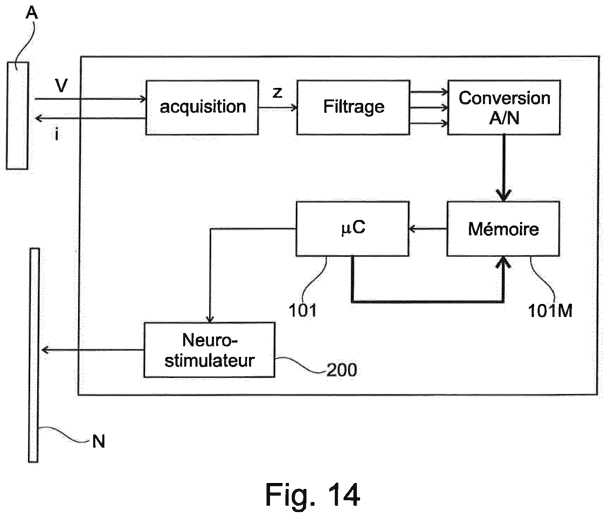

La Figure 14 reprend sous forme de blocs fonctionnels les différents composants mis en oeuvre dans un appareil de stimulation du nerf vague équipé du dispositif de mesure et d'analyse de bioimpédance selon la présente invention. Les données issues de la conversion analogique/numérique sont stockées dans une mémoire 101M associée au microcontrôleur 101, et les données issues de l'analyse fréquentielle décrite ci-dessus sont également stockées dans cette mémoire.

Une sortie du microcontrôleur est reliée à une entrée de commande d'un dispositif de commande de neurostimulation (200) situé dans le boitier implanté B, de manière à appliquer au nerf vague, via la sonde S disposée autour de lui, les impulsions de stimulation cardiaques appropriées en réponse à l'analyse des différents paramètres issus de la mesure d'impédance, effectuée par le microcontrôleur avec l'assistance éventuelle du processeur de traitement numérique.

En ce qui concerne les aspects logiciels opérés par le microprocesseur 101 d'analyse (ou le processeur 104) ou par un microprocesseur associé à la neurostimulation, l'invention peut être mise en oeuvre par une programmation appropriée du logiciel de commande d'un stimulateur connu, ici de type stimulateur cardiaque mais aussi, en variante, resynchroniseur, défibrillateur, etc. comprenant les signaux délivrés par l'analyse d'impédance et le cas échéant des moyens d'acquisition d'un signal fourni par des sondes endocavitaires.

L'invention peut ainsi s'appliquer à toute sonde implantée située dans un environnement où des variations d'impédance d'origine sanguine sont susceptibles d'être observées, soit dans un vaisseau sanguin tel que l'artère carotide, soit directement dans le coeur.

L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

On notera pour terminer que le procédé de l'invention est mis en oeuvre en partie par des moyens logiciels, au moyen d'algorithmes appropriés exécutés par un microcontrôleur ou un processeur numérique de signal. Pour la clarté de l'exposé, les divers traitements appliqués ont été décomposés et schématisés par un certain nombre de blocs fonctionnels distincts présentés sous forme de circuits interconnectés, mais cette représentation n'a toutefois qu'un caractère illustratif, ces circuits comprenant des éléments communs et pouvant correspondre en pratique à une pluralité de fonctions globalement exécutées par un même logiciel.

## Revendications

1. Un dispositif implantable, comprenant une sonde (S) de stimulation destinée à être placée dans une région corporelle à stimuler électriquement, ladite sonde comprenant :
- une série d'électrodes de stimulation (Ea, Eb) reliées à un dispositif de commande ; et
- une série d'électrodes (E1, E2, E3) aptes à effectuer des mesures de bioimpédance locale sur un flux sanguin (A) situé dans ladite région, et alignées parallèlement à une direction axiale de la sonde (S), comprenant :
- une électrode de référence (E1), une électrode d'injection de courant (E3) et une électrode de mesure (E2) située entre l'électrode de référence (E1) et l'électrode d'injection de courant (E3) ; dont
- une première paire d'électrodes de mesure de bioimpédance locale (E1, E3) reliées à un générateur de courant (G) pour faire circuler un courant de façon contrôlée dans le vaisseau sanguin ; et
- une deuxième paire d'électrodes de mesure de bioimpédance locale (E1, E2) reliées à l'entrée d'un circuit d'amplification et de traitement (AS, 108-110), et configurées de manière à capter un signal à partir duquel peuvent être extraites lesdites mesures de bioimpédance locale, ledit signal étant engendré en réponse audit courant,
**caractérisé en ce que** la première paire d'électrodes de mesure de bioimpédance locale et la deuxième paire d'électrodes de mesure de bioimpédance locale partagent une électrode commune (E1) formant électrode de référence ; et
**en ce que** les électrodes de la deuxième paire d'électrodes de mesure de bioimpédance locale (E1, E2) sont agencées de manière à capter ledit signal à partir duquel peuvent être extraites lesdites mesures de bioimpédance locale de façon indépendante d'une impédance de contact au point d'injection dudit courant.

2. Le dispositif de la revendication 1, **caractérisé en ce que** les électrodes de mesure de bioimpédance (E1-E3) sont agencées de façon affleurante à la surface d'un corps (CS) de la sonde (S).

3. Le dispositif de la revendication 1, **caractérisé en ce que** le circuit d'amplification et de traitement comprend des moyens (109, 112-115) de séparation en bandes de fréquences.

4. Le dispositif de la revendication 3, **caractérisé en ce que** le circuit d'amplification et de traitement comprend des moyens (101) aptes à déterminer des variations d'amplitude du signal dans les différentes bandes de fréquences.

5. Le dispositif de la revendication 3, **caractérisé en ce que** les bandes de fréquences sont des bandes de fréquences aptes à refléter au moins deux activités parmi une activité vasomotrice, une activité respiratoire, une activité hémodynamique et une activité du rythme cardiaque.

6. Le dispositif de la revendication 4, **caractérisé en ce qu'** une bande de fréquences est apte à refléter des variations de la pression sanguine sur un cycle cardiaque.

7. Le dispositif de la revendication 1, **caractérisé en ce que** la sonde (S) est agencée pour appliquer une neurostimulation au nerf vague, eten ce que les électrodes de mesure de bioimpédance (E1-E3) sont agencées pour longer un vaisseau (A) s'étendant généralement dans la même direction que le nerf vague.

8. Le dispositif de la revendication 7, **caractérisé en ce que** le vaisseau (A) est l'artère carotide, eten ce que les paires d'électrodes de mesure de bioimpédance (E1, E3 : E1, E2) sont séparées d'une distance comprise entre 10 et 25 mm environ au sein d'une paire.

9. Le dispositif de la revendication 8, **caractérisé en ce que** les électrodes de mesure de bioimpédance (E1-E3) ont une superficie comprise entre environ 0,5 et 2 mm².

10. Le dispositif de la revendication 8, **caractérisé en ce qu'** il comprend en outre un circuit de commande (200) apte à contrôler la neurostimulation en fonction des mesures de bioimpédance effectuées.

11. Le dispositif de la revendication 10, **caractérisé en ce qu'** il comprend un boitier implantable (B) relié à la sonde (S) par une liaison (L) comprenant des conducteurs pour la neurostimulation et des conducteurs pour les signaux de bioimpédance de la sonde vers le boitier.

## Patentansprüche

1. Implantierbare Vorrichtung, umfassend eine Stimulationssonde (S), die dazu bestimmt ist, in eine Körperregion eingesetzt zu werden, die elektrisch stimuliert werden soll, wobei die Sonde Folgendes umfasst:
- eine Reihe von mit einer Steuervorrichtung verbundenen Stimulationselektroden (Ea, Eb); und
- eine Reihe von Elektroden (E1, E2, E3), die dazu geeignet sind, lokale Bio-Impedanz-Messungen an einem in der Region befindlichen Blutstrom (A) durchzuführen und die parallel zu einer axialen Richtung der Sonde (S) ausgerichtet sind, umfassend:
- eine Referenzelektrode (E1), eine Stromeinspeisungselektrode (E3) und eine Messelektrode (E2), die sich zwischen der Referenzelektrode (E1) und der Stromeinspeisungselektrode (E3) befindet; darunter:
- ein erstes Paar von Elektroden zur Messung der lokalen Bio-Impedanz (E1, E3), die mit einem Stromgenerator (G) verbunden sind, um einen Strom gesteuert durch das Blutgefäß zu führen; und
- ein zweites Paar von Elektroden zur Messung der lokalen Bio-Impedanz (E1, E2), die mit einem Eingang einer Verarbeitungs- und Verstärkungsschaltung (AS, 108-110) verbunden sind, und die zur Erfassung eines Signals ausgebildet sind, aus dem die Messungen der lokalen Bio-Impedanz gewonnen werden können, wobei das Signal in Antwort auf den Strom erzeugt wird,
**dadurch gekennzeichnet, dass** das erste Paar von Elektroden zur Messung der lokalen Bio-Impedanz und das zweite Paar von Elektroden zur Messung der lokalen Bio-Impedanz eine gemeinsame Elektrode (E1) als Referenzelektrode teilen; und
dass die Elektroden des zweiten Paars von Elektroden zur Messung der lokalen Bio-Impedanz (E1, E2) derart angeordnet sind, dass sie das Signal erfassen, aus dem die Messungen der lokalen Impedanz unabhängig von einer Kontakt-Impedanz am Einspeisungspunkt des Stroms gewonnen werden können.

2. Vorrichtung nach Anspruch 1, **dadurch gekennzeichnet, dass** die Elektroden zur Messung der Bio-Impedanz (E1-E3) mit einer Oberfläche eines Körpers (CS) der Sonde (S) bündig angeordnet sind.

3. Vorrichtung nach Anspruch 1, **dadurch gekennzeichnet, dass** die Verarbeitungs- und Verstärkungsschaltung Mittel (109, 112-115) zur Trennung von Frequenzbändern umfasst.

4. Vorrichtung nach Anspruch 3, **dadurch gekennzeichnet, dass** die Verarbeitungs- und Verstärkungsschaltung Mittel (101) umfasst, die dazu geeignet sind, Variationen der Amplitude des Signals in den verschiedenen Frequenzbändern zu ermitteln.

5. Vorrichtung nach Anspruch 3, **dadurch gekennzeichnet, dass** die Frequenzbänder solche Frequenzbänder sind, die dazu geeignet sind, mindestens zwei Aktivitäten unter einer vasomotorischen Aktivität, einer Atmungsaktivität, einer hämodynamischen Aktivität und einer Herzrhythmus-Aktivität widerzuspiegeln.

6. Vorrichtung nach Anspruch 4, **dadurch gekennzeichnet, dass** ein Frequenzband dazu geeignet ist, die Variationen des Blutdrucks während eines Herzzyklus widerzuspiegeln.

7. Vorrichtung nach Anspruch 1, **dadurch gekennzeichnet, dass** die Sonde (S) zur Anwendung einer Neurostimulation am Nervus vagus angeordnet ist, und dass die Elektroden zur Messung der Bio-Impedanz (E1-E3) entlang eines Gefäßes (A) angeordnet sind, das sich im Wesentlichen in derselben Richtung wie der Nervus vagus erstreckt.

8. Vorrichtung nach Anspruch 7, **dadurch gekennzeichnet, dass** das Gefäß (A) die Kopfschlagader ist, und dass innerhalb eines Paars von Elektroden zur Messung der Bio-Impedanz (E1, E3 : E1, E2) die Elektroden um einen Abstand von ungefähr 10 bis 25 mm getrennt sind.

9. Vorrichtung nach Anspruch 8, **dadurch gekennzeichnet, dass** die Elektroden zur Messung der Bio-Impedanz (E1-E3) eine Oberfläche von ungefähr 0,5 bis 2 mm² aufweisen.

10. Vorrichtung nach Anspruch 8, **dadurch gekennzeichnet, dass** sie außerdem einen Steuerkreis (200) umfasst, die dazu geeignet ist, die Neurostimulation in Abhängigkeit der durchgeführten Bio-Impedanz-Messungen zu steuern.

11. Vorrichtung nach Anspruch 10, **dadurch gekennzeichnet, dass** sie ein implantierbares Gehäuse (B) umfasst, das mit der Sonde (S) über eine Verbindung (L) verbunden ist, die Leiter zur Neurostimulation und Leiter zur Leitung der Bio-Impedanz-Signale von der Sonde zum Gehäuse umfasst.

## Claims

1. An implantable device comprising a stimulation probe (S) designed to be placed in a body region that is to be stimulated electrically, said probe comprising:
- a series of stimulation electrodes (Ea, Eb) connected to a control device; and
- a series of electrodes (E1, E2, E3) adapted to carry out local bio-impedance measurements on a blood flow (A) in said region, and aligned in parallel with an axial direction of the probe (S), comprising:
- a reference electrode (E1), a current injection electrode (E3) and a measurement electrode (E2) located between the reference electrode (E1) and the current injection electrode (E3); including
- a first pair of local bio-impedance measurement electrodes (E1, E3) connected to a current generator (G) for circulating a current in a controlled manner inside the blood vessel; and
- a second pair of local bio-impedance measurement electrodes (E1, E2) connected to the input of a processing and amplification circuit (AS, 108-110) and configured to capture a signal from which said local bio-impedance measurements can be extracted, said signal being generated in response to said current,
**characterized in that** the first pair of local bio-impedance measurement electrodes and the second pair of local bio-impedance measurement electrodes share a common electrode (E1) as reference electrode; and
**in that** the electrodes of the second pair of local bio-impedance measurement electrodes (E1, E2) are arranged so as to capture said signal from which said local bio-impedance measurements can be extracted independently of a contact impedance at the injection point of said current.

2. The device of claim 1, **characterized in that** the bio-impedance measurement electrodes (E1-E3) are arranged flush with the surface of a body (CS) of the probe (S).

3. The device of claim 1, **characterized in that** the processing and amplification circuit comprises frequency band separation means (109, 112-115).

4. The device of claim 3, **characterized in that** the processing and amplification circuit comprises means (101) adapted to determine amplitude variations of the signal in the various frequency bands.

5. The device of claim 3, **characterized in that** the frequency bands are frequency bands that are adapted to reflect at least two activities among a vasomotor activity, a respiratory activity, a hemodynamic activity and a heart rate activity.

6. The device of claim 4, **characterized in that** a frequency band is adapted to reflect variations in the blood pressure during a single cardiac cycle.

7. The device of claim 1, **characterized in that** the probe (S) is arranged to apply nerve stimulation to the vagus nerve, and **in that** the bio-impedance measurement electrodes (E1-E3) are arranged so as to follow a vessel (A) extending substantially in the same direction as the vagus nerve.

8. The device of claim 7, **characterized in that** the vessel (A) is the carotid artery and **in that**, within both pairs of bio-impedance measurement electrodes (E1, E3 : E1, E2), the electrodes are separated by a distance of approximately 10 to 25 mm.

9. The device of claim 8, **characterized in that** the bio-impedance measurement electrodes (E1-E3) have a surface area of approximately 0.5 to 2 mm².

10. The device of claim 8, **characterized in that** it further comprises a control circuit (200) adapted to control the nerve stimulation according to the performed bio-impedance measurements.

11. The device of claim 10, **characterized in that** it comprises an implantable housing (B) connected to the probe (S) by a link (L) comprising conductors for nerve stimulation and conductors for conducting the bio-impedance signals from the probe to the housing.
